# EUROPÄISCHE PATENTSCHRIFT

(11) **EP 3 217 901 B1**
(45) Veröffentlichungstag und Bekanntmachung des Hinweises auf die Patenterteilung: **16.10.2019**
(21) Anmeldenummer: 15732667.9
(22) Anmeldetag: 29.06.2015
(51) Int. Cl.: A61B 17/70

(54) **VORRICHTUNG ZUM VERSPANNEN VON WIRBELN DER MENSCHLICHEN WIRBELSÄULE**
DEVICE FOR BRACING VERTEBRAS OF THE HUMAN SPINAL COLUMN
DISPOSITIF POUR LA MISE SOUS CONTRAINTE DES VERTÈBRES DE LA COLONNE VERTÉBRALE HUMAINE

(30) Priorität: 12.11.2014 DE 102014223112
(43) Veröffentlichungstag der Anmeldung: 20.09.2017
(73) Patentinhaber: Gebr. Brasseler GmbH & Co. KG, 32657 Lemgo (DE)
(72) Erfinder: KÜLLMER, Michael, 32657 Lemgo (DE); HAGEMANN, Frank, 32657 Lemgo (DE)
(74) Vertreter: Hoefer & Partner Patentanwälte mbB
(86) Internationale Anmeldenummer: PCT/EP2015/064685
(87) Internationale Veröffentlichungsnummer: WO 2016/074808

(56) Entgegenhaltungen:
- EP-A1- 1 808 141
- FR-A1- 2 950 243
- US-A1- 2003 187 433
- US-A1- 2006 217 716
- US-A1- 2012 035 670
- US-A1- 2012 083 850
- US-A1- 2012 165 875

## Beschreibung

Aus der DE 92 02 745 U1 ist eine Vorrichtung zum Verspannen von Wirbeln der menschlichen Wirbelsäule mit mindestens zwei Pedikelschrauben bekannt, deren ringförmiger Kopf einen zur Öffnung hin durchgehenden Schlitz mit Innengewindeabschnitten aufweist. In dem Schlitz ist eine Sicherungsschraube einschraubbar, welche einen in den Schlitz im Kopf der Pedikelschraube einlegbaren Stab sichert.

Die WO 94/26191 zeigt eine Osteosynthese-Vorrichtung mit einer Knochenschraube, insbesondere einer Pedikelschraube, mit einem eine Nut aufweisenden Gabelkopf und einem in der Nut des Gabelkopfes gelagerten Korrekturstab, welcher durch eine Strukturierung seiner Außenfläche drehgesichert ist.

Eine weitere derartige Vorrichtung zeigt die DE 692 06 318 T2. Weiterhin wird auf die WO 93/11715 A1, die WO 92/20294 A1, die WO 91/16020 A1 und die DE 92 02 745 U1 verwiesen. Ferner ist aus der US 2012/0083850 A1 eine Vorrichtung gemäß dem Oberbegriff des Anspruchs 1 bekannt. Ferner zeigt die US 2012/0035670 A1 eine Vorrichtung mit einem mehrteiligen Aufbau.

Bei derartigen Vorrichtungen zum Verspannen von Wirbeln, welche intern, im Körper des Patienten eingesetzt werden, ist es erforderlich, Maßnahmen zu treffen, dass die vom Operateur vorgewählten Einstellungen der Vorrichtung dauerhaft beibehalten werden und sich nicht durch Bewegungen des Körpers ändern können.

Der Erfindung liegt die Aufgabe zugrunde, eine Vorrichtung der eingangs genannten Art zum Verspannen von Wirbeln der Wirbelsäule zu schaffen, welche bei einfachem Aufbau und einfacher, betriebssicherer Anwendbarkeit eine hohe Formstabilität aufweist und gegen Lockerungen oder Versetzungen einzelner Bauelemente gesichert ist.

Erfindungsgemäß wird die Aufgabe durch die Merkmalskombination des Anspruchs 1 gelöst, die Unteransprüche zeigen weitere vorteilhafte Ausgestaltungen der Erfindung.

Die Erfindung zeigt somit eine Vorrichtung zum Verspannen von Wirbeln der menschlichen Wirbelsäule, mit zumindest einer Pedikelschraube, deren Kopf zumindest zum Teil sphärisch ausgebildet ist und in einem zumindest zum Teil sphärischen Lagerbereich eines Pedikelkopfes aufgenommen ist, wobei der Pedikelkopf eine zentrische Ausnehmung aufweist, welche den Lagerbereich umfasst, sowie einen quer zu der zentrischen Ausnehmung angeordneten Schlitz zur Aufnahme eines Stabs und einem dem Lagerbereich gegenüberliegend in der zentrischen Ausnehmung ausgebildeten Innengewinde, in welchem zur Klemmung des Stabs eine Sicherungsschraube angeordnet ist, wobei die Sicherungsschraube an ihrer dem Stab zugewandten Seite mit einer Verdrehsicherung versehen ist.

Die Erfindung sieht somit eine Konstruktion vor, bei welcher eine Sicherungsschraube in einem Pedikelkopf eingeschraubt wird, um einen Stab zu klemmen. Der Stab ist in einem Schlitz des Pedikelkopfes aufgenommen und wird mittels der Sicherungsschraube gegen das Ende des Schlitzes des Pedikelkopfes vorgespannt.

Bei Bewegungen der Wirbelsäule kann es vorkommen, dass sich die Sicherungsschraube lockert und somit der Stab nicht mehr ausreichend an dem Pedikelkopf und damit an der Pedikelschraube befestigt ist. Um dies zu verhindern, ist die erfindungsgemäße Verdrehsicherung vorgesehen. Diese umfasst in bevorzugter Ausgestaltung der Erfindung an der Stirnseite der Sicherungsschraube im Wesentlichen radial angeordnete Erhebungen und konvexe Bereiche. Die Sicherungsschraube verrastet somit auf der Oberfläche des Stabes, wodurch ein unbeabsichtigtes Lösen der Sicherungsschraube verhindert wird. Erfindungsgemäß erfolgt somit ein formschlüssiger Eingriff zwischen der stirnseitigen Fläche der Sicherungsschraube und der Oberfläche des Stabs. Diese formschlüssige Verrastung ist beim Einschrauben der Sicherungsschraube bei entsprechender Krafteinwirkung und unter Berücksichtigung der Elastizität der verwendeten Materialien problemlos möglich. Im montierten Zustand liegt jedoch eine Vorspannung vor, welche ein unbeabsichtigtes Drehen der Sicherungsschraube durch die formschlüssige Verrastung zwischen der stirnseitigen Fläche und dem Stab zuverlässig verhindert.

Die stirnseitige Fläche der Sicherungsschraube kann erfindungsgemäß in unterschiedlicher Ausgestaltung mit den Erhebungen und konkaven Bereichen versehen sein, beispielsweise in mehrfacher Teilung, so dass um den Umfang der Stirnseite, bezogen auf die Drehachse der Sicherungsschraube sternförmig radiale Erhebungen und radiale konkave Bereiche ausgebildet sind. Die Strukturierung dieser Erhebungen und konkaven Bereiche können abgerundet ausgebildet sein, es ist jedoch auch möglich, diese mit einem trapezförmigen Querschnitt, mit einem wulstartigen Querschnitt, abgeschrägt, oder mit einem dreieckigen Querschnitt zu versehen.

Um eine sichere Verrastung und Verdrehsicherung mit der Sicherungsschraube zu erzielen, ist in günstiger Weiterbildung der Erfindung vorgesehen, dass der Stab an seiner Oberfläche glatt oder aufgeraut oder strukturiert ausgebildet ist. Bei einer glatten Ausgestaltung kann der Stab sicher in die formschlüssigen radialen Erhebungen oder radialen konkaven Bereiche der Sicherungsschraube eingreifen. Bei einer aufgerauten Ausgestaltung der Oberfläche ergibt sich eine zusätzliche Reibkraft, die zusätzlich oder ergänzend zu der formschlüssigen Verrastung mit der Sicherungsschraube führt. Weiterhin ist es möglich, den Stab nicht rund auszubilden und beispielsweise profilierte oder abgeflachte Bereiche an seiner Oberfläche auszubilden.

Im Folgenden wird die Erfindung anhand von Ausführungsbeispielen in Verbindung mit der Zeichnung beschrieben. Dabei zeigt:
- Fig. 1: eine perspektivische Ansicht einer erfindungsgemäßen Vorrichtung im montierten Zustand,
- Fig. 2: eine Detailansicht eines montierten Zustands gemäß Fig. 1,
- Fig. 3, 4: Schnittansichten im montierten Zustand, analog Fig. 2,
- Fig. 5, 6: eine Seiten-Schnittansicht sowie eine stirnseitige Ansicht eines Ausführungsbeispiels der erfindungsgemäße Sicherungsschraube,
- Fig. 7, 8: eine Axial-Schnittansicht sowie Seitenansichten eines Ausführungsbeispiels einer erfindungsgemäßen Sicherungshülse,
- Fig. 9, 10: eine Axial-Teilschnittansicht sowie eine Seitenansicht und eine stirnseitige Schnittansicht eines erfindungsgemäßen Pedikelkopfes,
- Fig. 11: eine Seitenansicht sowie eine Schnittansicht einer erfindungsgemäßen Pedikelschraube,
- Fig. 12-18: perspektivische sowie stirnseitige Ansichten unterschiedlicher Ausgestaltungsbeispiele der erfindungsgemäßen Sicherungsschraube, und
- Fig. 19-25: perspektivische Darstellungen unterschiedlicher Ausgestaltungsformen des erfindungsgemäßen Stabes.

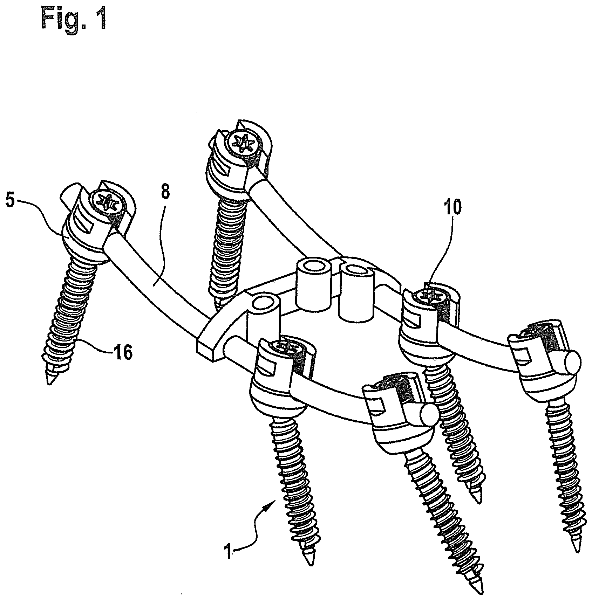

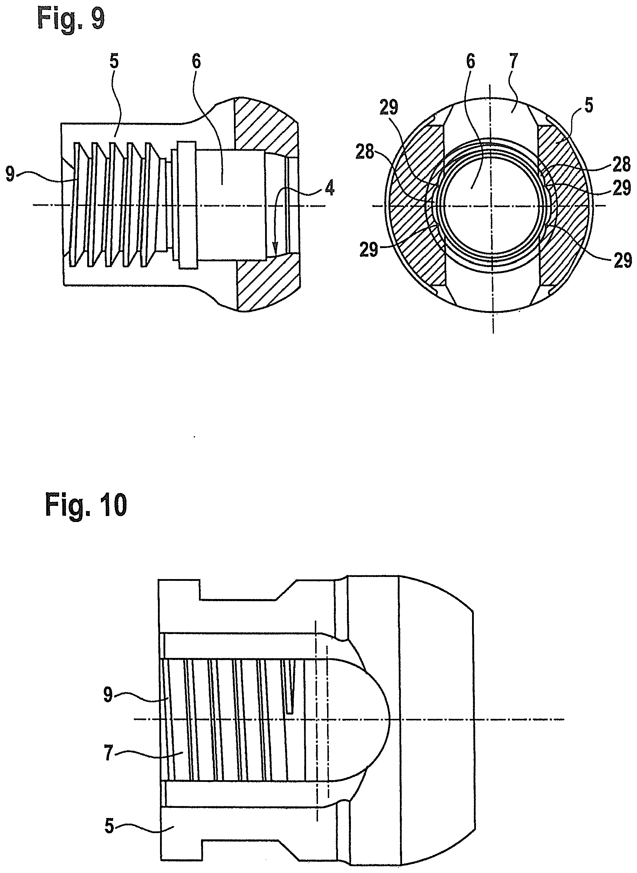

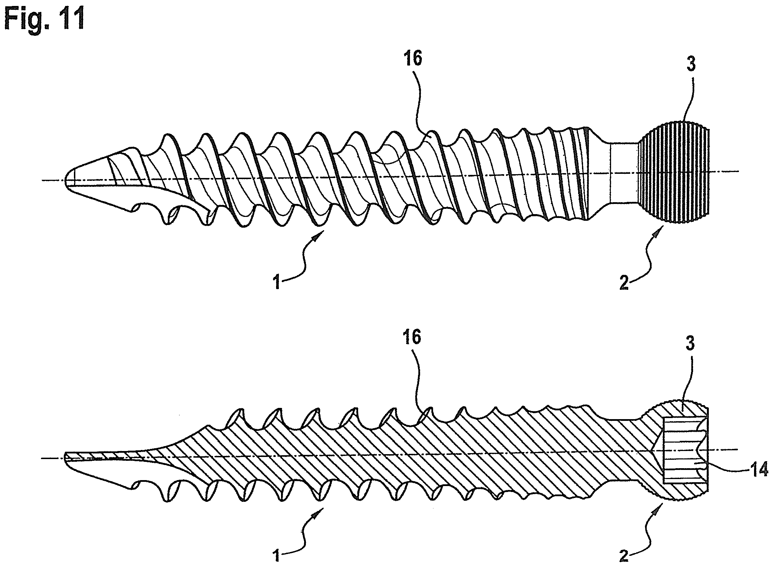

Die Fig. 1 zeigt eine perspektivische Darstellung einer erfindungsgemäßen Vorrichtung im montierten Zustand. Dabei sind mehrere Pedikelschrauben 1 gezeigt, die in unterschiedlichen Richtungen orientiert sind, so wie sich dies bei einem Einschrauben in eine Wirbelsäule darstellt. Die Pedikelschrauben sind mit einem Gewinde 16 versehen, so wie dies insbesondere nachfolgend in Verbindung mit Fig. 11 beschrieben wird. An ihrem oberen Ende weisen die Pedikelschrauben einen Kopf 2 auf (s. Fig. 11), welcher mit einem sphärischen Bereich 3 versehen ist. Die Pedikelschrauben 1 sind jeweils durch eine zentrische Ausnehmung eines Pedikelkopfes 5 geführt. Der Pedikelkopf 5 weist einen Schlitz 7 auf, in welchen ein Stab 8 eingelegt ist. Dieser wird mittels einer Sicherungsschraube 10 geklemmt, deren Gewinde in ein Innengewinde 9 des Pedikelkopfes 5 eingreift. Das Innengewinde 9 ist in einer zentrischen Ausnehmung 6 (s. Fig. 9) des Pedikelkopfes 5 ausgebildet. Durch die erfindungsgemäße Ausbildung der Sicherungsschraube 10 ergibt sich eine formschlüssige Verrastung mit dem Stab 8, so wie dies nachfolgend im Einzelnen erläutert wird.

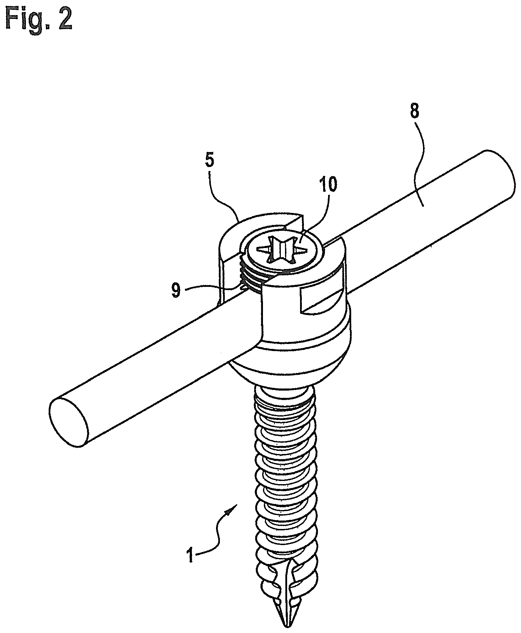

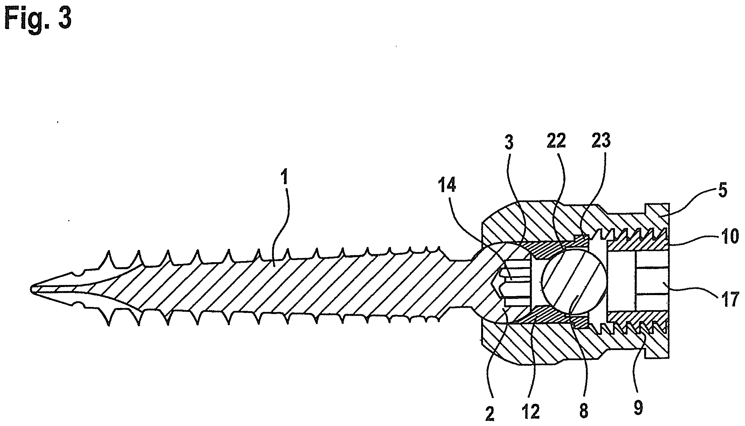

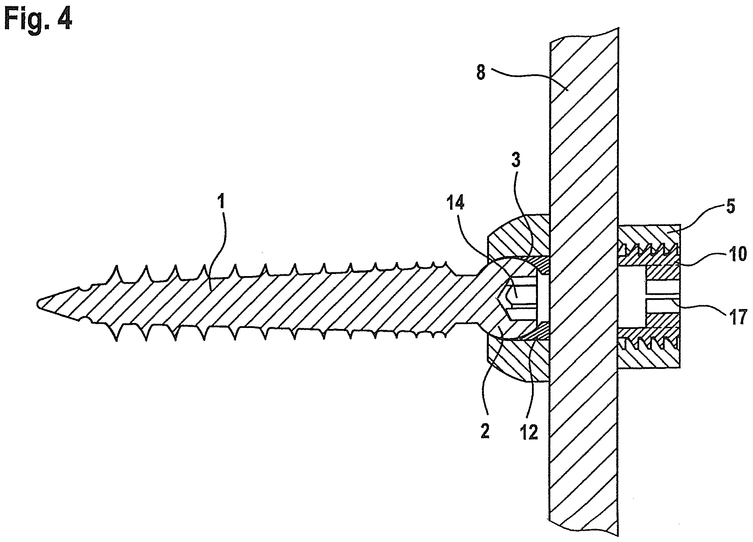

Die Fig. 2 zeigt eine vergrößerte perspektivische Darstellung im montierten Zustand. Aus den Darstellungen der Fig. 3 und 4, welche jeweils einen Axialschnitt längs der Längsachse der Pedikelschraube 4 zeigen, ergibt sich die Zuordnung der einzelnen Bauteile im montierten Zustand, wobei die Ansicht der Fig. 3 und 4 jeweils um 90° zueinander gedreht sind.

Die Pedikelschrauben 1 weisen an ihrem Kopf 2 eine Werkzeugeingriffsausnehmung 14 auf, s. auch Fig. 11. Das Gewinde ist als Kompressionsgewinde ausgebildet und weist eine variable Steigung im konischen Kerndurchmesser sowie gleichbleibende scharfe Gewindespitzen über die gesamte Schraubenlänge auf. Dies ergibt sich auch aus der Darstellung der Fig. 11. Der sphärische Bereich 3 des Kopfes 2 ist an seiner Oberfläche aufgeraut, beispielsweise durch umlaufende Rillen oder ähnliches.

Die Pedikelschraube 1, sowie insbesondere deren Kopf 2, sind durch die zentrische Ausnehmung 6 des Pedikelkopfes 4 durchgeführt bzw. durchgesteckt. Die zentrische Ausnehmung 6 des Pedikelkopfes 4 ist mit einem sphärischen Lagerbereich 4 versehen, welcher passend zu dem sphärischen Bereich 3 des Kopfes 2 der Pedikelschraube 1 ausgebildet ist. Somit liegt der sphärische Bereich 3 in flächigem Kontakt an dem sphärischen Lagerbereich 4, wodurch im geklemmten Zustand Relativbewegungen zwischen dem Pedikelkopf 5 und der Pedikelschraube 1 verhindert werden. Die Ausgestaltung des Pedikelkopfes 5 ergibt sich insbesondere auch aus den Fig. 9 und 10. Aus der stirnseitigen Ansicht der Fig. 9 (rechte Darstellung gemäß Fig. 9) ist der Schlitz 7 des Pedikelkopfes 5 deutlich ersichtlich.

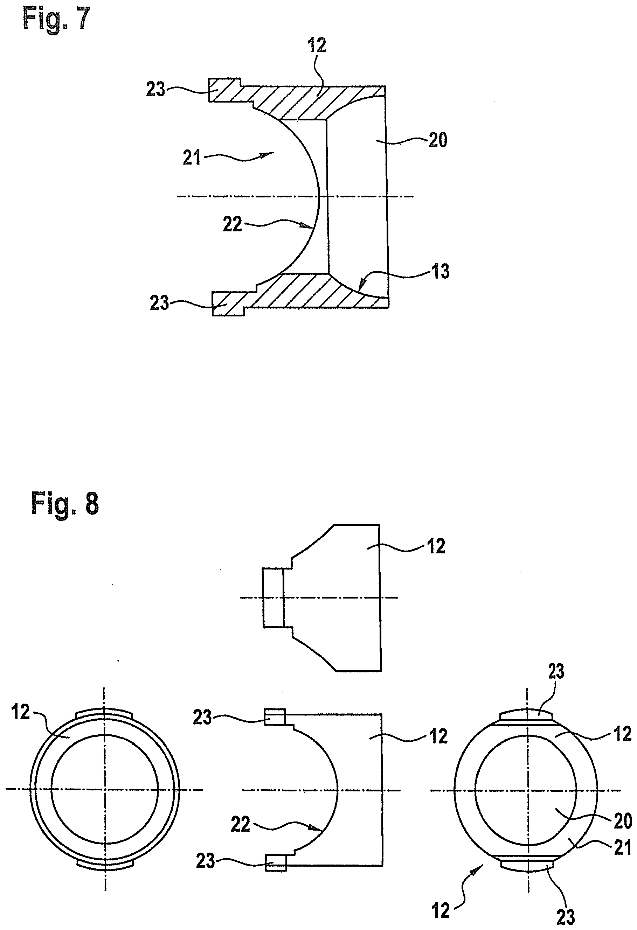

Angrenzend an den Kopf 2 der Pedikelschraube 1 ist in der zentrischen Ausnehmung 6 eine Sicherungshülse 12 angeordnet, welche nachfolgend im Zusammenhang mit den Fig. 7 und 8 beschrieben werden wird.

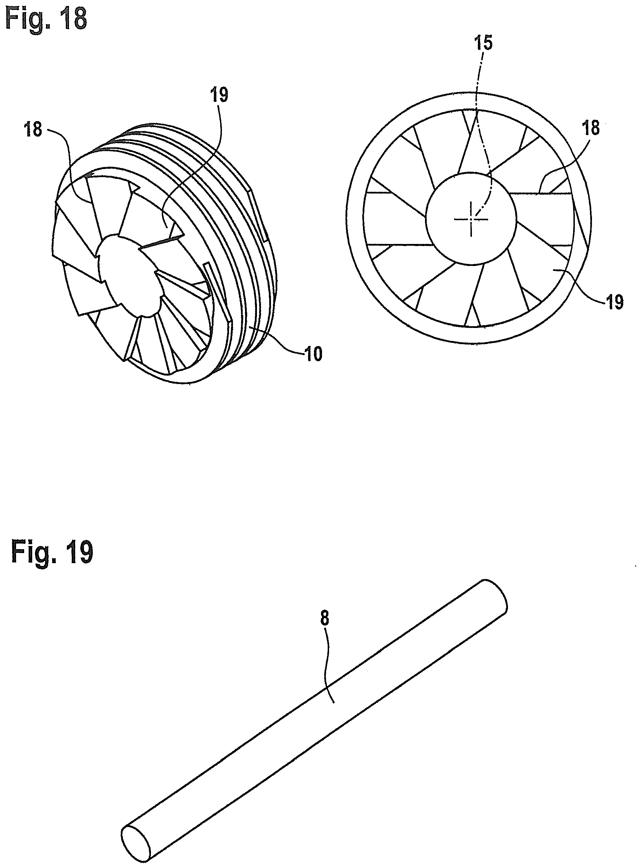

In dem Schlitz 7 ist ein Stab 8 eingelegt, so wie sich dies aus den Fig. 1 und 2 ergibt. Der Stab 8 wird nachfolgend im Zusammenhang mit den Fig. 19-25 näher erläutert.

Zur Klemmung des Stabes 8 dient die Sicherungsschraube 10, welche mit einer Werkzeugeingriffsausnehmung 17 versehen ist, beispielsweise einem Innenmehrkant oder einem Innensechsrund-Profil.

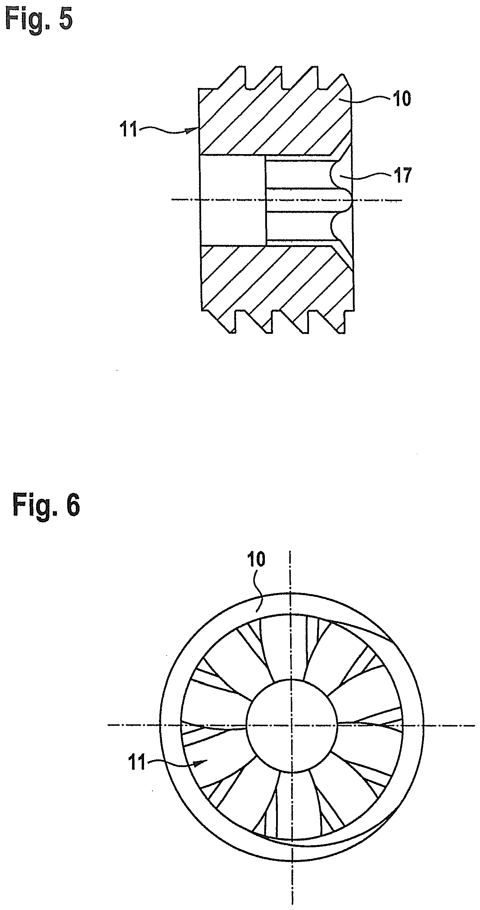

Die Sicherungsschraube 10 ist an ihrer Stirnseite, welche dem Stab 8 zugewandt ist, mit einer Verdrehsicherung 11 versehen. Diese umfasst alternierend angeordnet radiale Erhebungen 18 und radiale konkave Bereiche 19. Es ergibt sich somit eine sternartige Ausgestaltung der Stirnseite, s. insbesondere Fig. 6 und 12 bis 18. Die radialen Erhebungen 18 und die radialen konkaven Bereiche 19 sind alternierend zueinander angeordnet und führen beim Einschrauben der Sicherungsschraube 10 zu einer formschlüssigen Verrastung mit dem Stab 8.

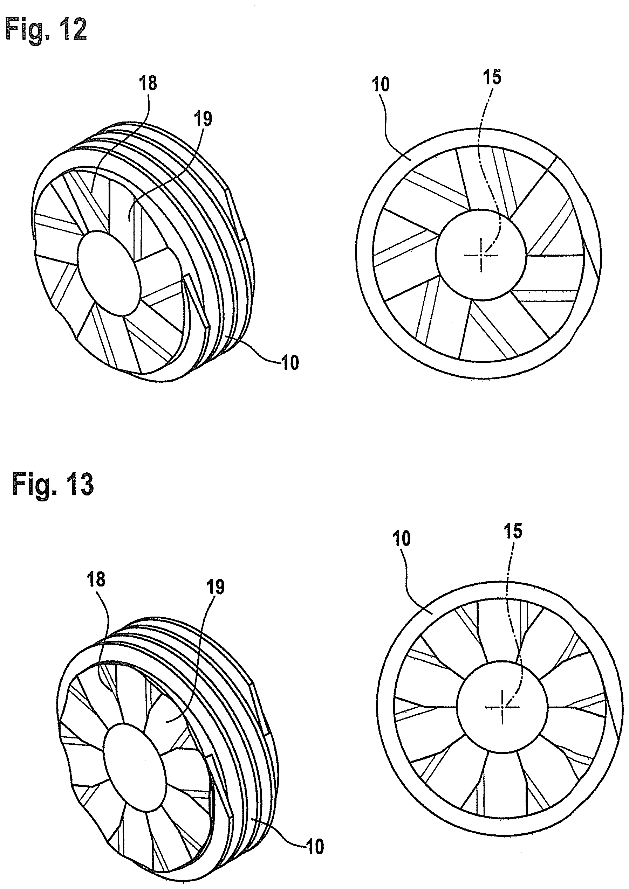

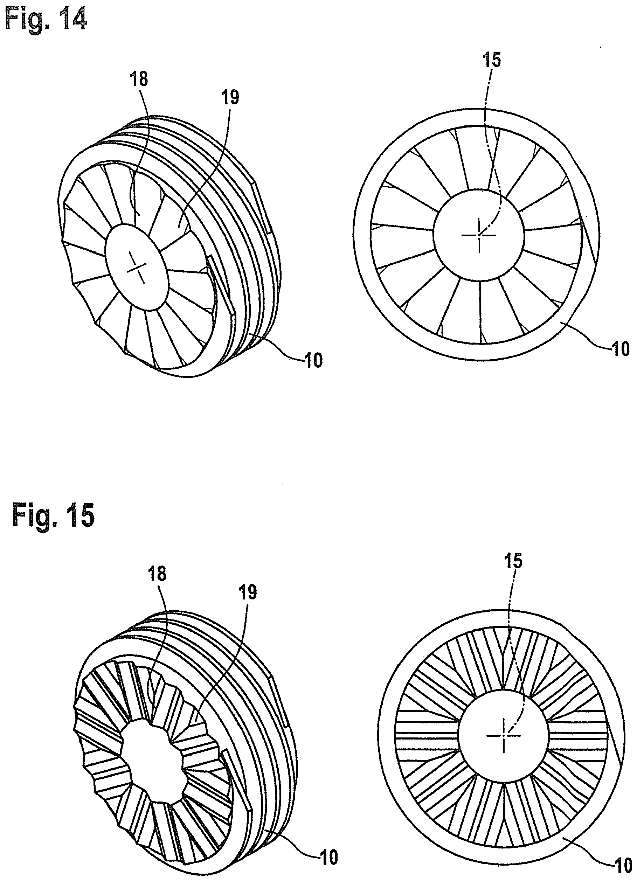

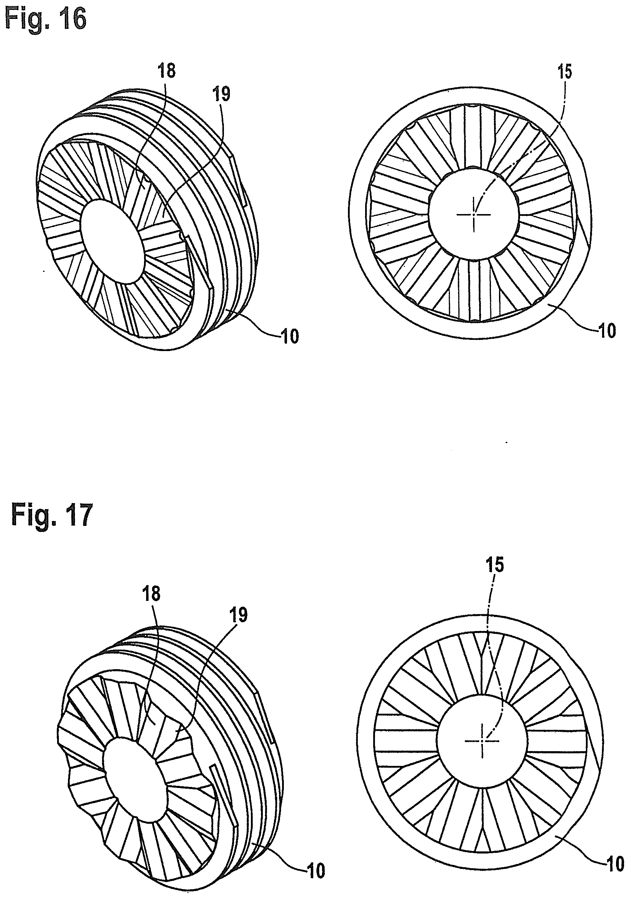

Wie sich aus den Fig. 6 und 12 bis 18 ergibt, kann die Verdrehsicherung 11 mit unterschiedlichen Teilungen ausgebildet sein. Die Fig. 12 bis 18 zeigen in der linken Darstellung jeweils eine perspektivische Ansicht und in der rechten Darstellung eine stirnseitige Ansicht, analog Fig. 6. In Fig. 12 ist eine 7er-Teilung vorgesehen, die Fig. 6 zeigt eine 10er-Teilung, ähnlich Fig. 13. In Fig. 14 ist eine 15er-Teilung vorgesehen. Die radialen Erhebungen 18 und die radialen konkaven Bereiche 19 können in unterschiedlicher Weise ausgebildet sein, so wie sich dies aus den Fig. 15-18 ergibt. Diese Ausführungsbeispiele zeigen die radialen Erhebungen bzw. radialen konkaven Bereiche 18, 19 in unterschiedlichen Querschnittsausgestaltungen, nämlich in Fig. 15 als doppeltes Dreieck, in Fig. 16 in einer wulstartigen Ausgestaltung, in Fig. 17 mit einem trapezförmigen Profil und in Fig. 18 mit einem abgeschrägten, im Querschnitt dreieckigen Profil. Bezogen auf die Drehung der Sicherungsschraube 10 um deren Drehachse 15 ergibt sich somit eine formschlüssige Verrastung nach der Befestigung der Sicherungsschraube 10, welche zu einem sicheren Klemmen des Stabes 8 führt. Dieser ist somit in dem Pedikelkopf 5 gesichert. Gleichzeitig führt die auf den Stab 8 durch die Sicherungsschraube 10 aufgebrachte axiale Vorspannung (bezogen auf den Pedikelkopf 5) zu einem Klemmen des Kopfes 2 der Pedikelschraube 1. Hierzu dient die in den Fig. 7 und 8 beschriebene Sicherungshülse 12.

Die Sicherungshülse 12 ist, wie sich auch aus Fig. 3 ergibt, in die zentrische Ausnehmung 6 des Pedikelkopfes 5 eingesetzt. Die Sicherungshülse 12 weist, wie in den Fig. 7 und 8 dargestellt, eine zentrische Ausnehmung 20 auf, in welche der Stab 8 einlegbar ist. Zugleich ist die Sicherungshülse 12 mit einem Schlitz 21 versehen, welcher mit einem im Wesentlichen zylindrischen Bereich 22 versehen ist. In den zylindrischen Bereich 22 ist der Stab 8 einlegbar, so dass dieser durch das Einschrauben der Sicherungsschraube 10 eine Druckkraft gegen die Sicherungshülse 12 ausübt. Der dem Kopf 2 der Pedikelschraube 1 zugewandte Bereich der Sicherungshülse 12 ist mit einem sphärischen Lagerbereich 13 versehen (s. Fig. 7), welcher passend zu dem sphärischen Bereich 3 des Kopfes 2 der Pedikelschraube 1 ausgebildet ist. Dies führt zu einer Klemmung des Kopfes 2 in den Pedikelkopf 5, so dass keine Relativbewegung zwischen dem Pedikelkopf 5 und dem Kopf 2 der Pedikelschraube 1 möglich ist.

Die Sicherungshülse 12 ist an ihren Endbereichen mit Rastnasen 23 versehen, welche in entsprechende Ausnehmungen 28 der zentrischen Ausnehmung 6 des Pedikelkopfes 5 einrasten, um den Pedikelkopf 5 nach dem Einschieben der Pedikelschraube 2 verliersicher an dieser zu haltern, bevor im weiteren Montagevorgang der Stab 8 montiert wird.

Wie sich aus einer Zusammenschau der Fig. 7, 8 und 3 ergibt, sind die Ausnehmungen 28 als Teil einer Ringnut ausgebildet, welche in der Wandung der zentrischen Ausnehmung 6 des Pedikelkopfes 5 ausgebildet ist. In diese Ringnut greifen die Rastnasen 23 ein. Durch Drehung der Sicherungshülse 12 um deren Mittelachse bzw. die zentrische Achse des Pedikelkopfes 5 können die Rastnasen 23 in die ringförmige Ausnehmung 28 eingedreht werden. Um ein Lockern der Rastnasen 23 und eine unbeabsichtigte Rückdrehung der Sicherungshülse 12 zu vermeiden, ist die Wandung der Ausnehmung 28 mit einer Strukturierung 29 versehen. Diese kann durch Bereiche unterschiedlichen Radiuses und damit unterschiedlicher Tiefe der Ringnut 28 gebildet sein. Somit verrasten die Rastnasen 23 bei einer Drehung längs der Ausnehmung 28 in den Strukturierungen 29. Die Sicherungshülse 12 ist somit gegen Drehung gesichert.

Wie sich insbesondere aus der Fig. 3 ergibt, ist die Sicherungshülse 12 mit einem axialen Spiel in dem Pedikelkopf 5 gelagert, sodass bei einem Einpressen des Stabes 8 mittels der Sicherungsschraube 10 die Sicherungshülse 12 in der zentrischen Ausnehmung 6 des Pedikelkopfes 5 gegen den sphärischen Lagerbereich 3 des Kopfes 2 der Pedikelschraube 1 vorgespannt werden kann, um den Kopf 2 innerhalb des Pedikelkopfes 5 verdrehsicher zu spannen.

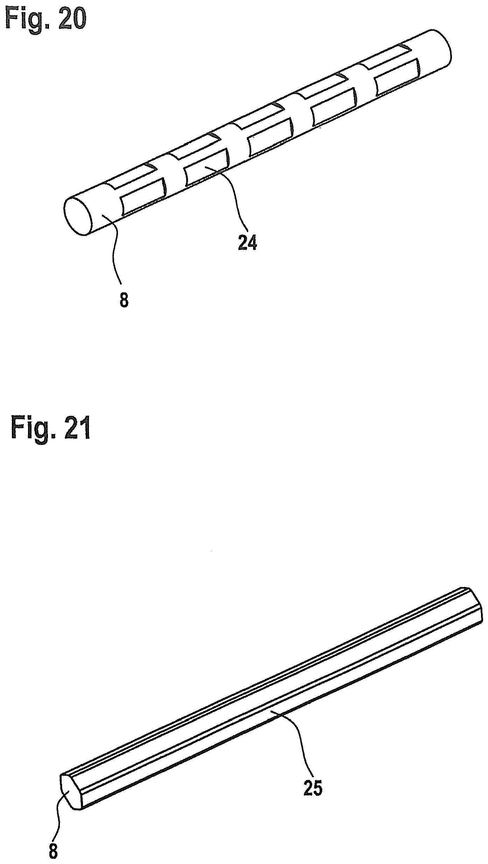

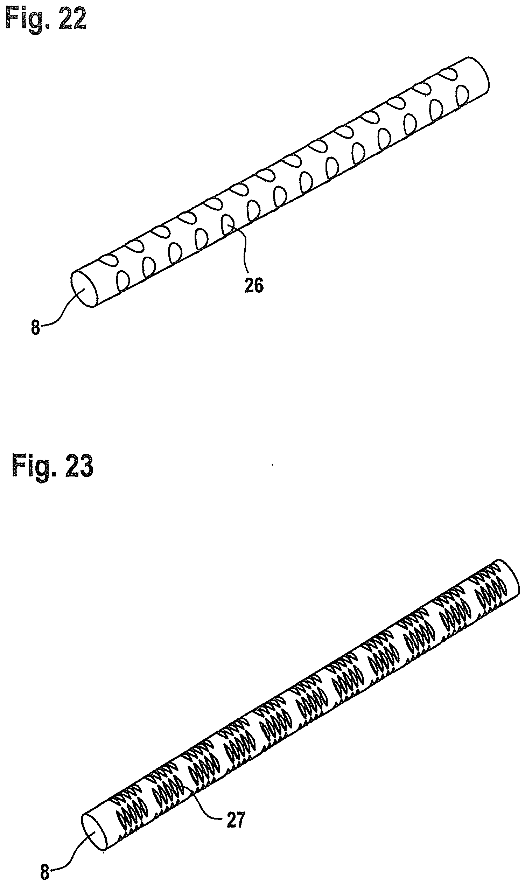

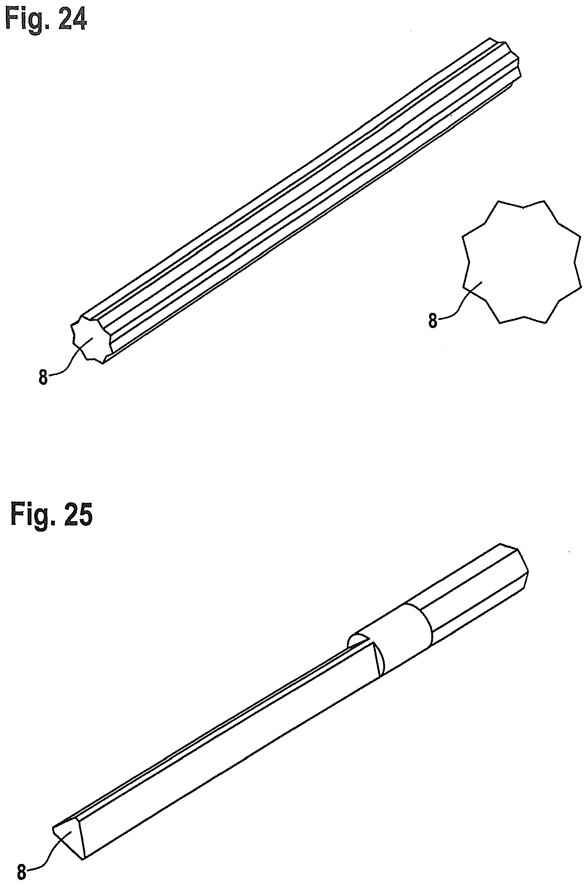

Die Fig. 19-25 zeigen unterschiedliche Ausgestaltungen des erfindungsgemäßen Stabes 8. Dieser kann glatt und zylindrisch ausgebildet sein (Fig. 19). Es ist jedoch auch möglich, einzelne abgeflachte Flächensegmente 24 vorzusehen, welche um den Umfang und in Längsrichtung des Stabes 8 verteilt angeordnet sind (Fig. 20). Die Fig. 21 zeigt eine Ausgestaltung mit durchgehenden Flächen 25. In Fig. 22 ist ein Ausführungsbeispiel gezeigt, bei welchem am Umfang des Stabes 8 sphärische Elemente 26 ausgebildet sind. Die Fig. 23 zeigt eine Modifikation, bei welcher gerillte Segmente 27 vorgesehen sind. Aus Fig. 28 ist ein Ausführungsbeispiel ersichtlich, bei welchem ein profilierter Querschnitt des Stabes 8 vorgesehen ist, s. rechte Bildhälfte von Fig. 24. Die Fig. 25 zeigt ein Ausführungsbeispiel, bei welchem unterschiedliche Querschnitte vorgesehen sind, abhängig vom Einsatzort des Stabes 8. Weiterhin ist es erfindungsgemäß möglich, lasermodifizierte Oberflächenstrukturen in beliebiger Ausgestaltung vorzusehen.

### Bezuaszeichenliste:

- 1: Pedikelschraube
- 2: Kopf
- 3: sphärischer Bereich
- 4: sphärischer Lagerbereich
- 5: Pedikelkopf
- 6: zentrische Ausnehmung
- 7: Schlitz
- 8: Stab
- 9: Innengewinde
- 10: Sicherungsschraube
- 11: Verdrehsicherung
- 12: Sicherungshülse
- 13: sphärischer Lagerbereich
- 14: Werkzeugeingriffsausnehmung
- 15: Drehachse
- 16: Gewinde
- 17: Werkzeugeingriffsausnehmung
- 18: radiale Erhebung
- 19: radialer konkaver Bereich
- 20: zentrische Ausnehmung
- 21: Schlitz
- 22: zylindrischer Bereich
- 23: Rastnase
- 24: Flächensegment
- 25: Fläche
- 26: sphärisches Element
- 27: gerilltes Segment
- 28: Ausnehmung
- 29: Strukturierung

## Patentansprüche

1. Vorrichtung zum Verspannung von Wirbeln der menschlichen Wirbelsäule, mit zumindest einer Pedikelschraube (1), deren Kopf (2) zumindest zum Teil sphärisch (3) ausgebildet ist und in einem zumindest zum Teil sphärischen Lagerbereich (4) eines Pedikelkopfes (5) aufgenommen ist, wobei der Pedikelkopf (5) eine zentrische Ausnehmung (6) aufweist, welche den Lagerbereich (4) umfasst, sowie einen quer zu der zentrischen Ausnehmung (6) angeordneten Schlitz (7) zur Aufnahme eines Stabs (8) und ein dem Lagerbereich (4) gegenüberliegend in der zentrischen Ausnehmung (6) ausgebildeten Innengewinde (9), in welchem zur Klemmung des Stabs (8) eine Sicherungsschraube (10) angeordnet ist, wobei die Sicherungsschraube (10) an ihrer dem Stab (8) zugewandten Seite mit einer Verdrehsicherung (11) versehen ist, **dadurch gekennzeichnet, dass**
eine formschlüssige Verrastung zwischen der Verdrehsicherung (11) an der Sicherungsschraube (10) und dem Stab (8) vorhanden ist, und
in der zentrischen Ausnehmung (6) des Pedikelkopfes (5) eine Sicherungshülse (12) angeordnet ist, welche mittels eines sphärischen Lagerbereichs (13) gegen den Kopf (2) der Pedikelschraube (1) anliegt und diese axial in dem Pedikelkopf (5) sichert, wobei die Sicherungshülse (12) mit dem Pedikelkopf (5) verrastet ist.

2. Vorrichtung nach Anspruch 1, **dadurch gekennzeichnet, dass** die Verdrehsicherung (11) in Form von radialen Erhebungen (18) und radialen konkaven Bereichen (19) ausgebildet ist.

3. Vorrichtung nach Anspruch 1 oder 2, **dadurch gekennzeichnet, dass** die dem Stab (8) zugewandte stirnseitige Seite der Sicherungsschraube (10) mit im Querschnitt wulstartig gerundeten radialen, bezogen auf eine Drehachse (15) der Sicherungsschraube (10), Erhebungen versehen ist.

4. Vorrichtung nach Anspruch 1 oder 2, **dadurch gekennzeichnet, dass** die dem Stab (8) zugewandte stirnseitige Seite der Sicherungsschraube (10) mit im Querschnitt trapezförmigen radialen, bezogen auf eine Drehachse (15) der Sicherungsschraube (10), Erhebungen versehen ist.

5. Vorrichtung nach Anspruch 1 oder 2, **dadurch gekennzeichnet, dass** die dem Stab (8) zugewandte stirnseitige Seite der Sicherungsschraube (10) mit im Querschnitt abgeschrägten radialen, bezogen auf eine Drehachse (15) der Sicherungsschraube (10), Erhebungen versehen ist.

6. Vorrichtung nach Anspruch 1 oder 2, **dadurch gekennzeichnet, dass** die dem Stab (8) zugewandte stirnseitige Seite der Sicherungsschraube (10) mit im Querschnitt dreieckigen radialen, bezogen auf eine Drehachse (15) der Sicherungsschraube (10), Erhebungen versehen ist.

7. Vorrichtung nach einem der Ansprüche 1 bis 6, **dadurch gekennzeichnet, dass** der Kopf (2) der Pedikelschraube (1) mit einer durch die zentrische Ausnehmung des Pedikelkopfes (5) und durch die Sicherungshülse (12) zugänglichen Werkzeugeingriffsausnehmung (14) versehen ist.

8. Vorrichtung nach einem der Ansprüche 1 bis 7, **dadurch gekennzeichnet, dass** der sphärische Bereich (3) des Kopfes (2) der Pedikelschraube (1) an seiner Oberfläche strukturiert und/oder aufgeraut ausgebildet ist.

9. Vorrichtung nach einem der Ansprüche 1 bis 8, **dadurch gekennzeichnet, dass** der Stab (8) an seiner Oberfläche glatt oder aufgeraut ausgebildet oder mit Flächen versehen ist.

## Claims

1. Device for bracing vertebrae of the human spinal column, having at least one pedicle screw (1) with a head (2) that is formed in a spherical (3) manner at least partially and that is received inside an at least partially spherical mounting area (4) of a pedicle head (5), wherein the pedicle head (5) has a centric recess (6) which comprises the mounting area (4) and a slit (7) for receiving a rod (8) that is arranged transversally with respect to the centric recess (6), as well as an internal thread (9) which is formed inside the centric recess (6) opposite the mounting area (4) and inside of which a safety screw (10) is arranged for the purpose of clamping the rod (8), wherein the safety screw (10) is provided with an anti-rotation device (11) at that side that is facing towards the rod (8), **characterized in that**
a form-fit meshing between the anti-rotation device (11) at the safety screw (10) and the rod (8) is provided, and
**in that** a safety sleeve (12) is arranged inside the centric recess (6) of the pedicle head (5), adjoining the head (2) of the pedicle screw (1) with a spherical mounting area (13) and axially securing the pedicle screw inside the pedicle head (5), wherein the safety sleeve (12) is preferably clamped to the pedicle head (5).

2. Device according to claim 1, **characterized in that** the anti-rotation device (11) is embodied in the form of radial elevations (18) and radial concave areas (19).

3. Device according to claim 1 or 2, **characterized in that** the front-end side of the safety screw (10) that is facing towards the rod (8) is provided with radial elevations - as viewed with respect to the rotational axis (15) of the safety screw (10) - that have a cross-section which is rounded in a bulge-like manner.

4. Device according to claim 1 or 2, **characterized in that** the front-end side of the safety screw (10) that is facing towards the rod (8) is provided with radial elevations - as viewed with respect to the rotational axis (15) of the safety screw (10) - that have a trapezoid cross-section.

5. Device according to claim 1 or 2, **characterized in that** the front-end side of the safety screw (10) that is facing towards the rod (8) is provided with radial elevations - as viewed with respect to the rotational axis (15) of the safety screw (10) - that have a chamfered cross-section.

6. Device according to claim 1 or 2, **characterized in that** the front-end side of the safety screw (10) that is facing towards the rod (8) is provided with radial elevations - as viewed with respect to the rotational axis (15) of the safety screw (10) - that have a triangular cross-section.

7. Device according to one of the claims 1 to 6, **characterized in that** the head (2) of the pedicle screw (1) is provided with a tool meshing recess (14) that is accessible through the centric recess of the pedicle head (5) and through the safety sleeve (12).

8. Device according to one of the claims 1 to 7, **characterized in that** the spherical area (3) of the head (2) of the pedicle screw (1) is formed so as to be structured and/or roughened up at its surface area.

9. Device according to one of the claims 1 to 8, **characterized in that** the rod (8) is formed so as to be smooth or roughened up at its surface area, or **in that** it is provided with surfaces.

## Revendications

1. Dispositif de mise sous contrainte de vertèbres de la colonne vertébrale humaine, avec au moins une vis pédiculaire (1), dont la tête (2) est réalisée de manière au moins en partie sphérique (3) et est logée dans une zone de support (4) au moins en partie sphérique d'une tête pédiculaire (5), dans lequel la tête pédiculaire (5) présente un évidement central (6), lequel comprend la zone de support (4), ainsi qu'une fente (7) disposée transversalement à l'évidement central (6) destinée à loger une barre (8) et un filetage femelle (9), réalisé dans l'évidement central (6) à l'opposé de la zone de support (4), dans lequel est disposée une vis de blocage (10) destinée à serrer la barre (8), dans lequel la vis de blocage (10) est pourvue sur sa face tournée vers la barre (8) d'une sécurité anti-rotation (11),
**caractérisé en ce que**
un encliquetage par coopération de formes est présent entre la sécurité anti-rotation (11) sur la vis de blocage (10) et la barre (8), et
un manchon de blocage (12), lequel s'applique contre la tête (2) de la vis pédiculaire (1) au moyen d'une zone de support sphérique (13) et bloque celle-ci axialement dans la tête pédiculaire (5), est disposé dans l'évidement central (6) de la tête pédiculaire (5), dans lequel le manchon de blocage (12) est encliqueté avec la tête pédiculaire (5).

2. Dispositif selon la revendication 1, **caractérisé en ce que** la sécurité anti-rotation (11) est réalisée sous forme de bosses radiales (18) et de zones concaves radiales (19).

3. Dispositif selon la revendication 1 ou 2, **caractérisé en ce que** la face frontale, tournée vers la barre (8), de la vis de blocage (10) est pourvue de bosses radiales arrondies à la façon d'un bourrelet en section transversale, par rapport à un axe de rotation (15) de la vis de blocage (10).

4. Dispositif selon la revendication 1 ou 2, **caractérisé en ce que** la face frontale, tournée vers la barre (8), de la vis de blocage (10) est pourvue de bosses radiales trapézoïdales en section transversale, par rapport à un axe de rotation (15) de la vis de blocage (10).

5. Dispositif selon la revendication 1 ou 2, **caractérisé en ce que** la face frontale, tournée vers la barre (8), de la vis de blocage (10) est pourvue de bosses radiales biseautées en section transversale, par rapport à un axe de rotation (15) de la vis de blocage (10).

6. Dispositif selon la revendication 1 ou 2, **caractérisé en ce que** la face frontale, tournée vers la barre (8), de la vis de blocage (10) est pourvue de bosses radiales triangulaires en section transversale, par rapport à un axe de rotation (15) de la vis de blocage (10).

7. Dispositif selon l'une quelconque des revendications 1 à 6, **caractérisé en ce que** la tête (2) de la vis pédiculaire (1) est pourvue d'un évidement d'insertion d'outil (14) accessible à travers l'évidement central de la tête pédiculaire (5) et à travers le manchon de blocage (12).

8. Dispositif selon l'une quelconque des revendications 1 à 7, **caractérisé en ce que** la zone sphérique (3) de la tête (2) de la vis pédiculaire (1) est réalisée de façon structurée et/ou rendue rugueuse sur sa surface.

9. Dispositif selon l'une quelconque des revendications 1 à 8, **caractérisé en ce que** la barre (8) est réalisée de façon lisse ou rendue rugueuse sur sa surface ou pourvue de faces.
